# EUROPEAN PATENT SPECIFICATION

(11) **EP 0 587 571 B1**
(45) Date of publication and mention of the grant of the patent: **27.05.1998**
(21) Application number: 92906577.9
(22) Date of filing: 06.03.1992
(51) Int. Cl.: C07K 14/575, C12N 15/12, A61K 38/22, A61K 38/17

(54) **HUMAN GALANIN, cDNA CLONES ENCODING HUMAN GALANIN AND A METHOD OF PRODUCING HUMAN GALANIN**
HUMANES GALANIN, CDNA-KLONE DIE FÜR HUMANES GALANIN KODIEREN UND EINE METHODE HUMANES GALAMIN HERZUSTELLEN
GALANINE HUMAINE, DES CLONES ADNc CODANT POUR LA GALANINE HUMAINE ET PROCEDE DE PRODUCTION DE GALANINE HUMAINE

(30) Priority: 06.03.1991 AU 4953/91
(43) Date of publication of application: 23.03.1994
(73) Proprietor: GARVAN INSTITUTE OF MEDICAL RESEARCH, Darlinghurst, NSW 2010 (AU)
(72) Inventor: EVANS, Helen, Frances, Bondi Junction, NSW 2022 (AU); SHINE, John, Woolwich, NSW 2110 (AU)
(74) Representative: Daniels, Jeffrey Nicholas
(86) International application number: AU9200097
(87) International publication number: WO9215681

(56) References cited:
- WO-A-92/12997
- WO-A-92/15015
- J.BIOL.CHEM., vol.262, no.35, 1987, USA pages 16755 - 58 M.E.VRONTAKIS ET AL. 'Isolation and Characterization of a Complementary DNA (Galanin) Clone from Estrogen- induced Pitutary Tumor Messenger RNA'
- PROC. NATL. ACAD. SCI. USA, vol.85, February 1988 pages 1065 - 69 L.M.KAPLAN ET AL. 'Tissue- specific expression of the rat galanin gene'
- FEBS LETTERS, vol.234, no.2, July 1988 pages 400 - 406 A.RÖKAEUS ET AL. 'Nucleotide sequence analysis of cDNAs encoding a bovine galanin precursor protein in the adrenal medulle and chemical isolation of bovine gut galanin'
- PROC. NATL. ACAD. SCI. USA, vol.83, September 1986 pages 6287 - 91 A.RÖKAEUS ET AL. 'Construction of a porcine adrenal medullary cDNA library and nucleotide sequence analysis of two clones encoding a galanin precursor'
- Endocrinology, Vol. 129, No. 3, 1991, pp 1682-1684, H.F. EVANS and J. SHINE, "Human Galanin: Molecular cloning reveals a Unique Structure".
- Neuroscience Letters, Vol. 136, 1992, pp 105-108, L.G. ULMAN et al., "Effects of human, rat and porcine galanins on cardiac vagal action and blood pressure in the anaesthetised cat".
- Proc. Natl. Acad. Sci. (USA), Vol. 88, Dec. 1991, pp 11435-11439, W.E. SCHMIDT et al., "Isolation and primary structure of pituitary human galanin, a 30-residue nonamidated neuropeptide".
- FEBS Lett., Vol. 283, No. 2, June 1991, pp 189-194, M. BERSANI et al., "Human Galanin: primary structure and identification of 2 molecular forms".
- FEBS Lett., Vol. 234, No. 2, July 1988, pp 400-406, A. ROKAEUS and M. CARLQUIST, "Nucleotide sequence analysis of cDNAs encoding a bovine galanin precursor protein in the adrenal medulla and chemical isolation of bovine gut galanin".
- Proc. Natl. Acad. Sci. (USA), Vol. 85, February 1988, pp 1065-9, L.M. KAPLAN et al., "Tissue-specific expression of the rat galanin gene".
- Proc. Natl. Acad. Sci. (USA), Vol. 83, September 1986, pp 6287-6291, A. ROKAEUS and M.J. BROWNSTEIN, "Construction of a porcine adrenal medullary cDNA library and nucleotide sequence analysis of two clones encoding a galanin precursor".

## Description

### Field of the Invention

The present invention relates to a peptide having the amino acid sequence of human galanin as deduced from the nucleotide sequence of human preprogalanin cDNA. The present invention further relates to cDNA clones encoding the peptide. In addition, the present invention encompasses therapeutic uses of the peptide, and the use of the peptide in designing galanin antagonists and agonists.

### Background of the Invention

Galanin is a putative neuropeptide which was first isolated from porcine small intestine in 1983(1). Porcine galanin is a peptide of 29 amino acid residues which was named for its N-terminal glycine and amidated C-terminal alanine residues(1). The cDNAs encoding galanin have been cloned from three species, rat (2), porcine (3) and bovine (4), revealing that galanin is a proteolytic product of a larger precursor protein known as preprogalanin(2). Galanin shows 90% homology between the species but little similarity to other known peptides(1). Antibodies raised to porcine galanin have allowed the mapping of galanin-like-immunoreactivity (GAL-LI) to discrete regions of the Central Nervous System (CNS) and throughout the Peripheral Nervous System (PNS) of several other species including man.

Immunohistochemical mapping of GAL-LI in the CNS has been performed most intensively in the rat where the highest concentrations have been found in the median eminance and hypothalamus(5). These results are consistent with more recent in situ hybridisation studies where the localisation of preprogalanin in the rat brain tentatively suggests the involvement of galanin in the regulation of several factors ranging from water balance behaviour and feeding to blood pressure control (6). Similarly, radioimmunoassay of galanin in the baboon brain showed high GAL-LI in the hypothalamus and median eminance, and also GAL-LI in association with limbic structures such as the amygdala(7). Immunohistochemistry and in situ studies of preprogalanin mRNA during development of the rat has shown tissue specific sex differences in galanin concentration, notably in the anterior pituitary(5) where its expression is eostrogen dependent(9). The overall distribution of GAL-LI and its colocalisation in discrete neuronal cells with catecholamines, serotonin, GABA, acetylcholine and various other peptides (10) strongly suggest a modulatory role for galanin. A noteworthy example is the coexistence of galanin with acetylcholine in nerve fibers projecting from the basal forebrain to the hippocampus, in the rat (11) and baboon (7) which has led to speculation that galanin may play a role in Alzheimers disease. There is, however, conflicting evidence concerning the expression of galanin in this region of the human brain. Although the physiological role of galanin in the CNS has not yet been established its pharmacology suggests a role in neuroendocine regulation. Injection of galanin into the third ventricle of rats causes increased growth hormone(13) and injection into the paraventricular nucleus (PVP) enhances food intake(14).

In the PNS, distribution of GAL-LI suggests that galanin is widespread. Galanin distribution and its pharmacology, which is diverse and often species specific, both suggest a range of physiological actions for galanin. However, some confusion may have arisen as to its pharmacological role through the use of porcine galanin in experiments involving other species. In numerous mammalian species the highest concentrations of GAL-LI are found in the intestine(1), pancreas(15), adrenal glands (3), and respiratory(16) and genitourinary tracts (17). Galanin action on the pancreas and its possible role in diabetes is controversial; it has been established that porcine galanin infusion in dogs(15), and rat and porcine galanin perfusion through the isolated rat pancreas(18), decrease plasma insulin levels. However there are conflicting results concerning porcine galanin action on the pig pancreas(19). In the dog, galanin also decreases somatostatin while increasing glucagon but this may not be the case in other species(15). Intravenous porcine galanin causes growth hormone secretion in a variety of species including man. However, intravenous porcine galanin infusion in man at a concentration sufficiently high to elicit an increase in growth hormone levels, does not cause the expected inhibition of insulin secretion (20). The apparent discrepancy may be due to the difference in amino acid sequence of human verses porcine galanin, or it may be simply a reflection of the species-specific effects of galanin. Visualisation of GAL-LI in neurons innervating the islets of several species (15) added to a proposal to explain the galanin-induced inhibition of insulin secretion in rat B-cell lines (21) support a neuromodulatory role for galanin on endocrine pancreatic action. Other pharmacological effects of galanin in the PNS include the species-specific stimulatory or inhibitory action of galanin on the smooth muscle activity of several mammalian species (22).

Galanin receptors have been identified in a hamster insulin-secreting B-cell tumor (23), rat (24) and monkey brain (25), and smooth muscle membranes (22). The distribution of galanin binding correlates with that of GAL-LI and therefore supports the role of galanin in neurotransmission. It is not clear whether there are subtypes of the galanin receptor, nor which region of the peptide is responsible for binding to its receptor. Studies on the biological effect of tryptic fragments of galanin on smooth muscle preparations (22), in addition to auto-radiographic binding studies on RINm5F pancreatic B cell lines (26) and on intestinal membrane preparations (27), present conflicting results.

The molecular biology of the galanin gene has not yet been examined in humans. Porcine preprogalanin is a 123 amino acid residue protein that comprises a signal sequence, galanin (29 amino acids) and a 59 amino acid peptide known as galanin mRNA associated peptide (GMAP). The length and structure of rat porcine and bovine preprogalanin are similar. The 20% difference in galanin amino acid homology across the species is manifest over the C-terminal end of the peptide. The sequence in all species identified to date suggests post-translational cleavage of glycine extended galanin, followed by amidation. GMAP is also well conserved across the species, which has led to speculation that it is biologically active; it includes a region of 35 amino acids that shows 78% homology across the species and within this region a stretch of 17 residues that shows greater homology.

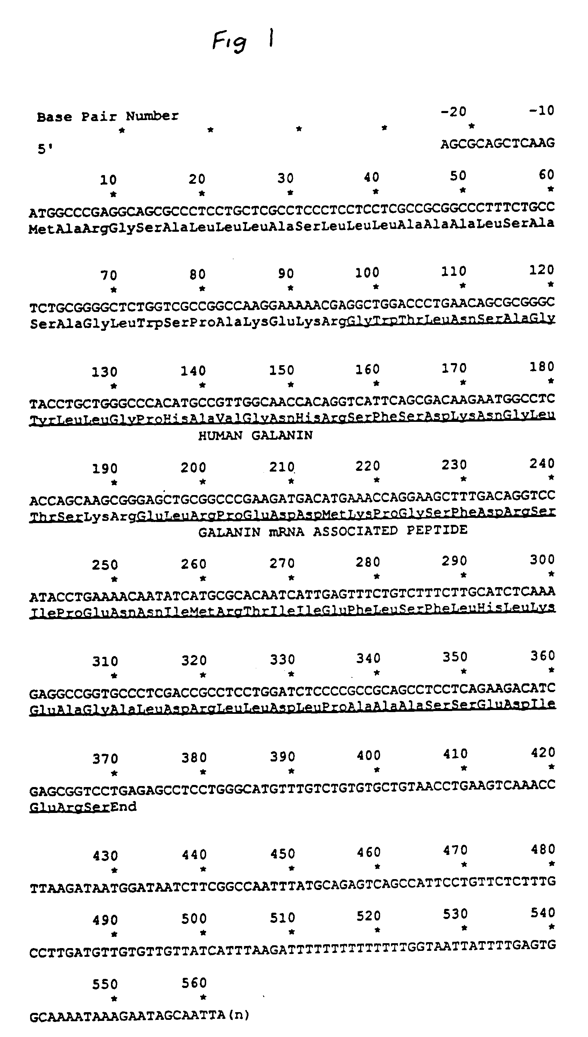

This invention discloses the isolation and characterisation of human preprogalanin from a neuroblastoma cell line cDNA library and from a pituitary cDNA library (28). Oligonucleotides complementary to two conserved regions of pig and rat preprogalanin were used in a polymerase chain reaction (PCR) to specifically amplify the corresponding sequence from neuroblastoma and pituitary cDNA. The two amplification oligonucleotides used (No. 1 and 2) correspond to amino acids 29-37 and 105-97 of rat and pig preprogalanin, respectively, and flank a 230 basepair region encoding galanin and the N-terminus of GMAP (Fig. 1) (29). An additional oligonucleotide (No. 3) within this region was used to probe for the correct PCR product (30). The amplified region from both sources of DNA was subcloned and then sequenced (31), revealing identical sequences. The region amplified from neuroblastoma cDNA was used as a probe to isolate clones encoding the complete preprogalanin cDNA from this library (32). Later, the pituitary cDNA library was screened with the same probe, in order to ascertain that any amino acid differences apparent between human preprogalanin and other species, were not due to the erroneous translation of DNA in the neuroblastoma cultured cell line.

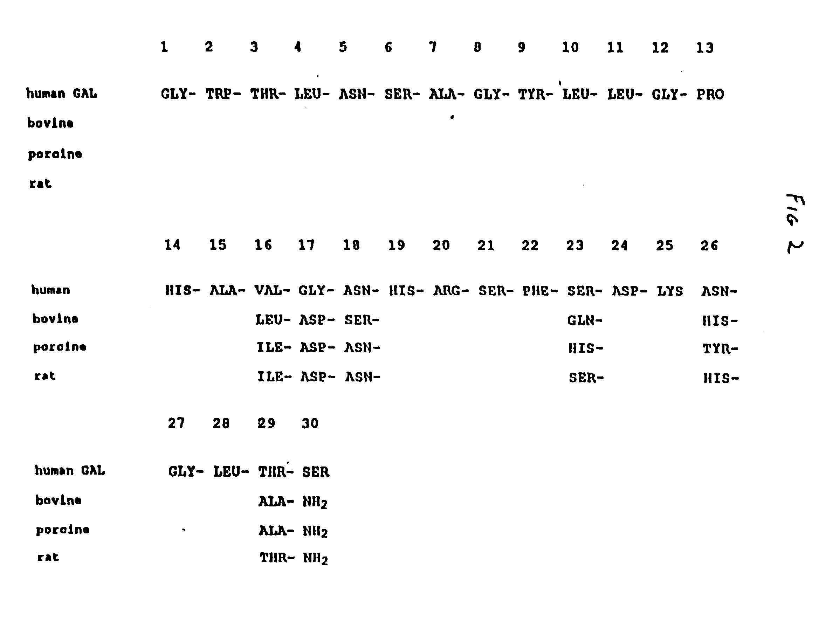

The primary structure of human preprogalanin cDNA clones isolated from both libraries were identical but different to that of pig, cow and rat. In general, amino acid substitutions only occurred at positions noted for variability amongst the other species (Fig. 2), thus confirming that galanin, and GMAP to a lesser extent, are both well conserved. However, several of these changes (e.g. 17, 23 and 26 in galanin) involve amino acids with very different physical and chemical properties suggesting that such changes are important for the correct function of human galanin. Also important is that human galanin is rendered unique by the striking substitution of a serine for a glycine residue at the C-terminus of galanin, directly proceeding the lys-arg cleavage site in the precursor protein. This implies that human galanin is not amidated at its C-terminus, in contrast to other species, where the glycine residue serves as an amide donor to the proceeding residue after proteolysis. Consequently, human galanin may have a variety of biological properties that differ from porcine, rat, and bovine galanin.

### Summary of the Invention

Accordingly, in a first aspect the present invention consists in a polypeptide having the following amino acid sequence:- GWTLNSAGYLLGPHAVGNHRSFSDKNGLTS or a functional equivalent thereof being achieved by conservative substitutions selected from the following:
G, A; V, I, L, M; D, E; N, Q; S, T; K, R, H; and F, Y, W.

In a preferred embodiment of the present invention the polypeptide fragment has the amino acid sequence:-

GWTLNSAGYLLGPHAVNHRSFSDKNGLTS

As used herein in relation to polypeptide sequences the term "functional equivalent" is intended to cover minor variations in the amino acid sequence which do not deleteriously affect the biological activity of the polypeptide. It will be recognised by those skilled in the art that a number of modifications may be made to the peptide of the present invention without deleteriously affecting the biological activity of the peptide. This may be achieved by various conservative substitutions in the peptide sequence where such substitutions do not substantially decrease the biological activity of the peptide. By conservative substitutions the intended combinations are:-
G, A; V, I, L, M; D, E; N, Q; S, T; K, R, H; and F, Y, W.

It may also be possible to add various groups to the peptide of the present invention to confer advantages such as increased potency or extended half-life in vivo without substantially decreasing the biological activity of the peptide. Peptides designed to perform these functions are described as galanin agonists. These additions and changes include the introduction of D-amino acid residues and the formation of cyclic analogues.

In a second aspect the present invention consists in a cDNA molecule encoding the peptide of the present invention, the cDNA molecule having a sequence substantially as shown in Figure 1 from nucleotide 97 to 186 or a functionally equivalent sequence.

In a third aspect the present invention consists in a DNA molecule encoding human preprogalanin and GMAP, the DNA molecule having a sequence substantially as shown in Figure 1 or a functionally equivalent sequence.

As used herein in relation to DNA sequences the term "functionally equivalent sequence" is intended to cover minor variations in the DNA sequence which, due to degeneracy in the DNA code, do not result in the sequence encoding different polypeptides.

Further, this term is intended to cover alterations in the DNA code which lead to changes in the encoded peptide, but in which such changes do not affect the biological activity of the peptide.

A different half life for human galanin in vivo can be expected, in addition to differences in binding affinity for specific receptors and thus potency between different species.

Of particular interest will be the effect of human galanin on insulin inhibition. Infusion of both porcine and rat galanin through an isolated rat pancreas showed that both types of galanin inhibited insulin and somatostatin release, although porcine galanin was less potent than rat galanin (18). In addition, rat galanin enhanced glucagon secretion whereas porcine galanin was ineffectual. The difference in activity of pig and rat galanin has been ascribed to the 4 amino, acid differences that exist between them at their C-terminus. Similarly, the 5 amino acids that differ between human and porcine galanin, coupled with the difference due to amidation, may be responsible for the lack of expected insulin inhibition observed when porcine galanin was infused into human subjects (20). Controversy concerning galanin action on the pancreas, and other examples of the species specific effect of galanin, such as the effect of galanin on the GI tract, indicate that it is preferable to use galanin homologous to the species under investigation.

In a fourth aspect the present invention consists in a method of producing human galanin comprising culturing a cell transformed with the cDNA molecule of the second or third aspect of the present invention under conditions which allow expression of the DNA sequence and recovering the human galanin.

While it is possible to form the polypeptide of the present invention by biological means involving recombinant techniques in prokaryotic or eucaryotic cells the polypeptides may also be formed by chemical synthesis. The decision as to which route of synthesis is used will depend primarily on the length of peptide to be synthesised.

From preliminary results it is clear that the polypeptide of the present invention has therapeutic application in modulating pancreatic activity, as a stimulator of growth hormone and as an attenuator of cardiac vagal function.

Accordingly, in further aspects the present invention consists in a method of modulating pancreatic activity, stimulating the production of growth hormone or attenuating cardiac vagal function in a human comprising administering the peptide of the present invention to the human.

The present invention also consists in the use of the polypeptide of the present invention in the preparation of a medicament for modulating pancreatic activity, stimulating the production of growth hormone or the attenuation of cardiac vagal function.

It has been demonstrated that galanin antagonists can be developed by chemical synthesis of chimeric galanin-like peptides. The N-terminal galanin fragment (amino acids 1-13), which binds the galanin receptor, can be coupled to a peptide of an α-helical structure that stabilises the N-terminal portion but has no innate biological action. The resulting chimera is described as a galanin antagonist, since it will bind but not activate the galanin receptor, thus inhibiting the action of endogenous galanin. The ability of such a chimeric peptide to function as a galanin antagonist can be assessed by measuring its binding efficiency to galanin receptors expressed in RINm5F cells and the ability to reverse the inhibited insulin response to galanin. A galanin antagonist will displace 125I-galanin binding from RINm5F cells in a dose dependent manner and reverse the inhibited glyceraldehyde-induced insulin response to galanin. The antagonist functions as a competitor to galanin but has no effect itself on glyceraldehyde-induced insulin secretion.

Using the polypeptide of the present invention it will be possible to screen peptides and other compounds for galanin agonist and antagonist activity. This would be done by receptors expressed in RINm5F cells. The compounds which showed competitive binding would then be assessed for biological activity.

In another aspect the present invention consists in a method of screening compounds for galanin agonist or antagonist activity comprising assessing the ability of the compound to compete with the peptide of the present invention for binding to cell receptors and assessing the biological activity of the compounds which competitively bind.

The present invention also relates to galanin antagonists obtained by this screening method.

In order that the present invention may be more clearly understood, preferred forms thereof will now be described with reference to the following examples and accompanying Figures in which:-
Fig. 1 shows the nucleotide sequence of preprogalanin, the amino acid sequence of human galanin and GMAP;
Fig. 2 provides a comparison of the amino acid sequence of human galanin with that of bovine, porcine, and rat;

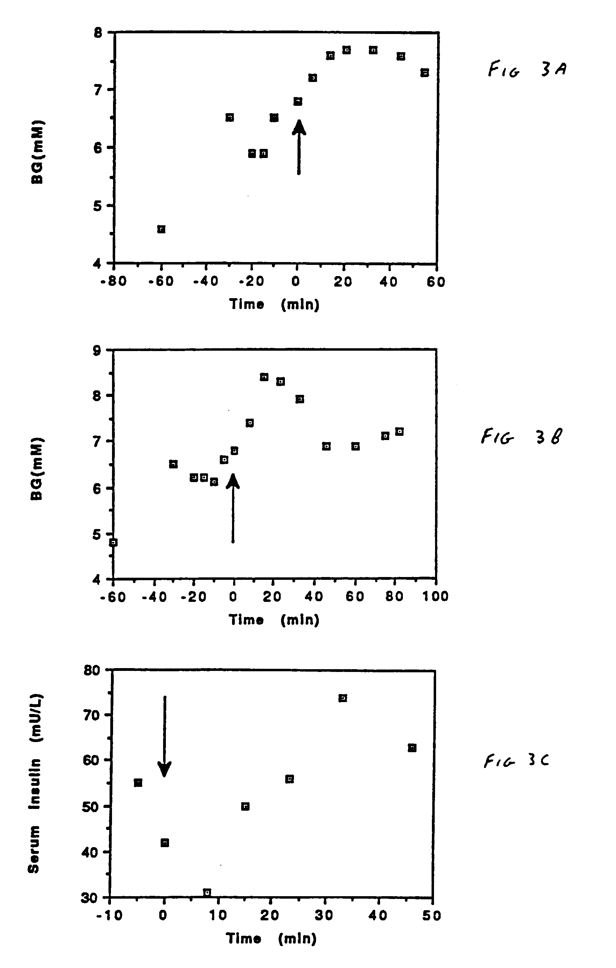

Fig. 3 shows the effect of administration of human galanin on levels of blood glucose (BG; (a) 350 µg and (b) 250 µg) and serum insulin levels (c 250 pg) in the conscious rat. The arrow indicates the time point at which the galanin was administered;
Fig. 4 shows the experimental protocol for infusion of human galanin into humans;
Fig. 5 shows the effect of administration of human galanin on pulse rate in a human subject.
Fig. 6 shows the effect of administration of human galanin on plasma glucose and insulin levels in a human subject ( ―•― saline; ―○― 1x10⁻⁹M human galanin ―□― 3-4 X10⁻⁹M human galanin; Arrows 1 and 3 show commencement and stoppage of galanin infusion, respectively and arrow 2 shows when glucose was administered).
Fig. 7 shows the effect of administration of human galanin or growth hormone levels in a human subject (―□― saline; ―•― 1x10⁻⁹M human galanin; ―■― 3-4 x 10⁻⁹M human galanin).

### EXAMPLES

### MATERIALS AND METHODS

### cDNA Libraries:

Two cDNA libraries were used for library screening and also as a source of template DNA for a polymerase chain reaction (PCR). The neuroblastoma cDNA library (Cat No. HL1007, Clontech Laboratories Inc., USA) contained 1.05 x 10⁶ independent clones inserted into a λgt10 vector at its EcoRI cloning site. The pituitary cDNA library was obtained from Dr P. Seeburg (Centre for Molecular Biology, University of Heidelberg, FRG) and was also carried in λgt10 and cloned in the EcoRI site.

### Oligonucleotide Synthesis:

With the exception of oligonucleotides directed to the EcoRI cloning site of λgt10 (Promega, VIC., Australia), all oligonucleotides were prepared on a DNA synthesiser (Applied Bio-systems, DNA synthesiser Model 380B, Burwood, Australia).
oligonucleotide sequence:

1.    GAATTCAAGGA(A/G)AAGAGAGGCTGGAC(T/C)CTGAA (EcoRI site
      incorporated)

2.   CCATAAGCTTGC(G/C)CC(G/C)GC(G/A/T/C)TCTTT(A/G)AG
     (A/G)TGCA(G/A)GAA (HindIII site incorporated).

3.   CCATAAGCTTAATGA(C/T)CTGTGG(C/T)TGTC(A/G)A(T/G)(C/G)
     GCATG (HindIII site incorporated)

### Polymerase chain reaction (PCR):

In order to prepare template cDNA, a plate lysate method was used to propagate phage (T. Maniatis, E.F. Fritsch, J. Sambrook, Molecular Cloning: A Lab Manual, 2Ed., Cold Spring Harbour Press, USA, 2.65 (1989)). The libraries were plated at 20,000 plaques per 150mm diameter plate and extracted into storage medium (SM=0.1M NaCl/0.008M MgSO₄7H₂O/0.05M Tris.HCl/0.02% gelatin) which gave a preparation with a titre of 1 x 10⁹ phage per ml. The phage stock (2ml) was extracted with phenol/CHCl₃ and the DNA was then precipitated with ethanol.

The PCR was used to amplify a 230 base pair region of preprogalanin from the neuroblastoma cDNA library (oligos 1 and 2) and also to amplify the cDNA library clones (λgt10 oligos) that were later isolated by screening. In both reactions, a Hybaid intelligent heating block (Model 1HB 2024, Hybaid, Middx., UK) was used with the following temperature parameters: hold at 95 C(5'), then 25 cycles of 92°C(1'), 42°C(1') and 72°C(1'). Each reaction contained KCl. (50mM), gelatin (100ug/ml), MgCl₂ (1.5mM), Tris-HCl (pH=8, 10mM), DNA (10ng-100ug), dNTP (200uM), Tth polymerase (0.25U, Toyobo, Japan) and oligonucleotides (500uM).

PCR products were separated on a 3% Nuseiveagarose gel (FMC Bioproducts, ME, USA) (products less than 500 basepairs) or 1% agarose (products greater 700 basepairs). The identity of PCR product bands was established by Southern blotting (E Southern, J. Mol. Biol, 98, 503 (1975)) the DNA onto a nylon membrane (Zeta probe, Bio-Rad Laboratories Inc., CA, USA) using 0.4M NaOH as the transfer buffer followed by hybridisation with oligo #3. Pre-hybridisation was performed at 42°C in a solution of 5 x SSPE (1X SSPE=0.18M Nacl.10mM NaH₂PO₄/1mM NaEDTA pH=7), 0.5% sodium dodecyl sulphate (SDS) and 5 x Denhardt's solution (1 x Denhardt's solution = 0.02% Ficoll-400/0.02% bovine serum albumin/0.02% polyvinylpyrolidone-40) and 100µg/ml heat denatured salmon sperm DNA. Hybridisation was performed in the same solution with the addition of probe for 6-12 hours at 42°C. The oligo was labelled with γ³²P ATP Amersham, International PLC, UK) using T4 polynucleotide kinase (BRL, MD, USA). Blots were washed at 37°C in 1 x SSC (1 x SSC=0.151M NaCl/0.1675 M tri-sodium citrate)/ 0.1% SDS prior to exposure to X-ray film (Kodak Eastman, NY, USA) at -70°C with an intensifying screen for 12 hours.

### Subcloning and sequencing:

PCR products were separated on a gel as described above. The appropriate band was excised and purified using GeneClean (Bio 101, CA, USA), before restriction digestion. The PCR product generated by preprogalanin oligos 1 and 2 was digested with HindIII and EcoRI, whereas the PCR product generated with the λgt10 oligos was digested with EcoRI, before ligation into M13mp19. The M13mp19 subclones were used to transform JM101 competent cells (Maniatis, 1.82-1.84) and subsequently single stranded DNA was prepared using standard methods (Maniatis, 4.29-4.30) for sequencing (Kit No. Q5800 Promega). Sequencing difficulties due to secondary structure were overcome with the use of Taq DNA polymerase (Kit No. Q 5540, Promega), as the sequencing enzyme at a reaction temperature of 70 °C.

### cDNA Library Screening:

The cDNA libraries described above were screened with the 230 base pair PCR product encoding the sequence of neuroblastoma preprogalanin. The probe was excised from a gel and purified using gene clean before labelling (25ng) with α³² dCTP in a random priming reaction (kit No. 8187SA, BRL). Approximately 6 x 10⁵ plaques were plated on 2YT plates (A3, Maniatis) lifted onto Hybond N nylon filters (Amersham) and fixed according to the manufacturer's recommendations. Pre-hydridisation and hybridisation were performed at 65°C as described above. Filters were washed to a stringency of 0.1% SDS /0.1% SSC at 65 °C and exposed overnight to X-ray film with itensifying screens.

### Blood Glucose and Secretion of Gluco-Regulatory Hormones The Administration of Human Galanin to Conscious Rats

Rats were maintained on established diets and cannulated under anaesthesia. The rats were then allowed to recover and infused with glucose. Ten minutes later a bolus dose of human galanin was administered and blood samples were taken over the next three hours. Following the sampling procedures the animals were sacrificed.

An elevation of blood glucose levels was observed in response to bolus administration of 350 µg (110 nmol; Fig. 2a) and 250 µg (80 nmol; Fig 2b) human galanin. the elevation of blood glucose levels in response to the administration of 250 µg (80 nmol in Fig. 2c) human galanin correlates with a drop in levels of circulating insulin.

### Growth Hormone Secretion

### Infusion of Human Galanin into Humans

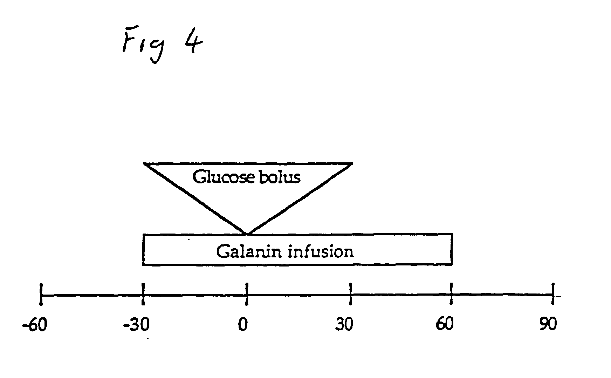

The experimental protocol for the infusion of human galanin into humans to achieve maximal circulating levels of galanin of approximately 3-4 x 10⁻⁹M is shown in Figure 4.

### The effect of human galanin on blood glucose and secretion of glucoregulatory hormones in humans

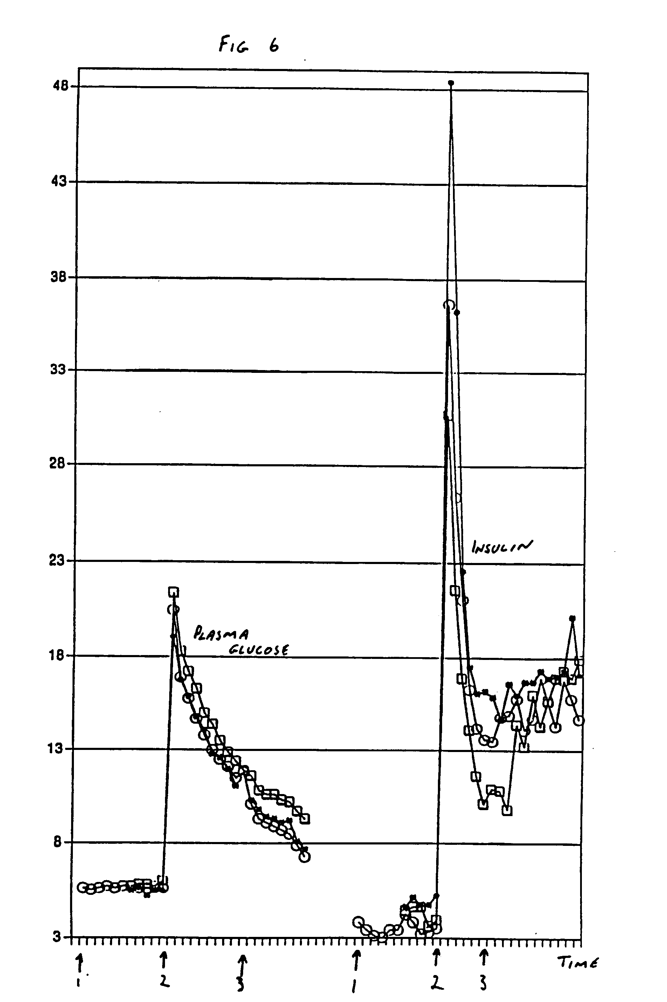

Preliminary data for one subject indicate that the administration of human galanin according to the protocol described in Fig. 4, to achieve maximal circulating levels of 1 x 10⁻⁹M and 4 x 10⁻⁹M human galanin, resulted in a detectable suppression of insulin secretion (Fig. 6; Y-Axis units : mIU/L). This was associated with an elevation of plasma glucose relative to the control situation (Fig. 6; Y-Axis units : mM).

### The effect of human galanin on growth hormone secretion

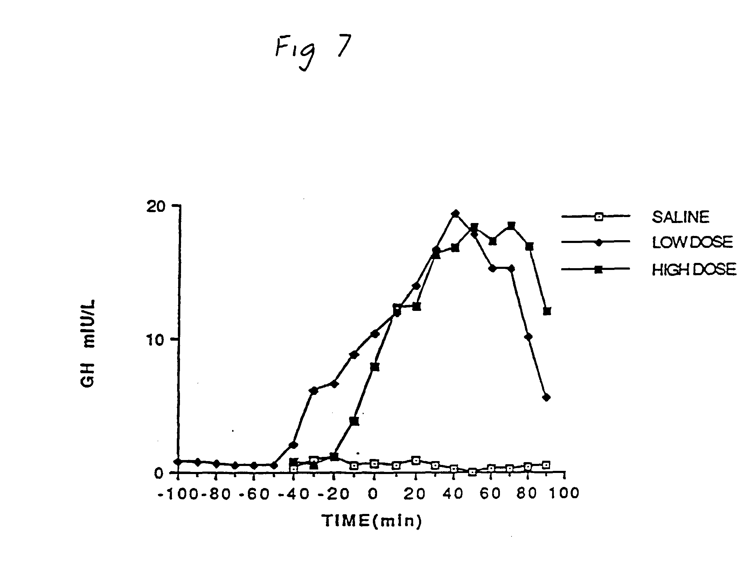

The administration of human galanin to human volunteers according to the protocol described in Fig. 4 resulted in an elevation of growth hormone levels at circulating levels of both 1 x 10⁻⁹M and 3-4 x 10⁻⁹ M human galanin in the two subjects studied to date. The effect of human galanin in one of these two subjects at the two dosage rates employed is shown in Fig. 7.

### Cardiovascular Effects

### The effect of human galanin on blood pressure and vagal nerve function in the anaesthetised cat

The intravenous injection of human galanin into anaesthetised cats resulted in an attenuation, of cardiac vagal slowing of heart rate.

### Cardiovascular effect of human galanin in humans

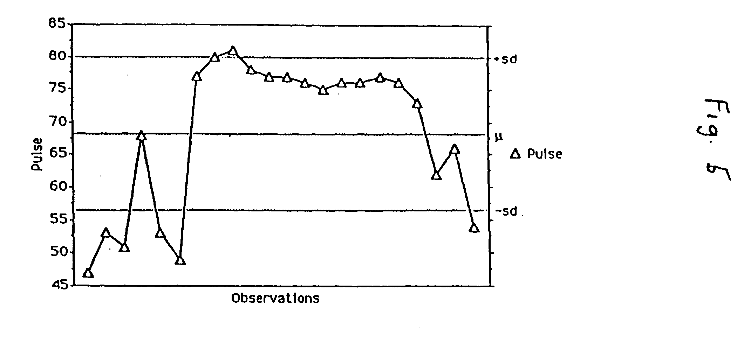

The infusion of human galanin into humans as detailed in Fig. 4 resulted in an increase in pulse rate (see Fig. 5), consistent with an effect of human galanin on attenuation of vagal function in the human.

From studies conducted to date it is believed that human galanin will have a number of therapeutic uses including:-
1. Inhibition of gastrointestinal activity, e.g. as an antidiarrhea agent.
2. Inhibition of insulin secretion e.g. modulate activity of endocrine pancreas in pancreatic disorders.
3. As a potent stimulator of growth hormone which acts independently of growth hormone releasing hormone.
4. As an attenuator of cardiac vagal function.
5. As yet not well characterised effects in the nervous system e.g. its depletion in Alzeimers disease, effects on appetite, prolactin release etc.

Experiments conducted using rat models support the first three uses, while studies in humans set out above with human galanin demonstrate the ability of human galanin to modulate insulin secretion, growth hormone secretion and cardiac vagal functions.

Prior to the present invention the possible therapeutic uses of galanin were largely restricted due to the species-specific pharmacological action of galanin i.e. exogenous human galanin when administered to humans would have a different pharmacological effect than commercially available pig and rat galanin. Prior to the present invention the reasons for this species specificity were not understood. It is now believed that the species specificity is due, at least in part, to the several amino acid striking differences between human galanin and that of other species and of particular note is that human galanin is not extended by glycine at its C-terminal, and that in its place is a serine residue which excludes the possibility of amidation after post-translational cleavage in vivo.

### REFERENCES

1. K. Tatemoto, A. Rokaeus, H. Jornvall, T.J. McDonald and V. Mutt, FEBS Lett. 164,124 (1983).

2. L.M. Kaplan, E.R. Spindel K.J. Isselbacher and W.W. Chin, Proc. Natl. Acad. Sci. U.S.A. 85, 1065 (1988).

3. A. Rokaeus and M.J. Brownstein, Proc. Natl. Acad. Sci. U.S.A., 83, 6287 (1983).

4. A. Rokaeus and M. Carlquist, FEBS Lett., 234, 400 (1988).

5. A. Rokaeus, T. Melander, T. Hokfelt, J.M. Lundberg, K. Tatemoto, M. Carlquist and V. Mutt, Neurosci. Lett, 47,161 (1984).

6. A.L. Gundlach, W. Wisden, B.J. Morris and S.P. Hunt, Neurosci. Lett., 114, 241 (1990).

7. M.F. Beal, S. M. Gabriel, K.J. Swartz, U.M. MacGarvey, Peptides, 9, 847 (1988).

8. S.M. Gabriel, L. M. Kaplan, J.B. Martin and J.I. Koenig, Peptides, 10, 369 (1989).

9. L.M. Kaplan, S.M. Gabriel, J.I. Koenig, M.E. Sunday, E.R. Spindel, B.J. Martin, W.W. Chin, Proc. Natl. Acad. Sci. U.S.A., 85, 7408 (1988).

10. T. Melander and W.A. Staines, Neurosci. Lett., 68,17 (1986): M.J. Brownstein, E. Mezey, Progress in Brain Research, Vol. 68, (EDS.T. Hokfelt, K. Fuxe and B. Pernow), 161-168, Elsevier.

11. T. Melander, W.A. Staines, T. Hokfelt, A. Rokaeus, F. Eckenstein, P.M. Salvaterra and B.H. Wainer, Brain Research, 360, 130 (1985).

12. V. Chan-Palay, J. Comp. Neurol. 243, 543 (1988): J.H. Kordower and E. J. Murfson, J. Comp. Neurol. 294,281 (1990).

13. A. Ottlecz, W.K. Sampson, S.M. McCann, Peptides, 51 (1986).

14. S.E. Kyrkoulii, B.G. Stanley and S.F. Leibowitz, Eur. J. Pharmacol. 122, 159 (1986).

15. B.E. Dunning, B. Ahren, R.C. Veith, G. Bottcher, F. Sundler, G.J. Taborsky, Am.J. Physiol. 251,14:E127 (1986) .

16. A. Cheung, J.M. Polak, F.E. Bauer, A. Cadieux, N.P. Christophides, D.R. Springall, S.R. Bloom, Thorax, 40, 889 (1985).

17. F.E. Bauer, N.D. Christophides, G.W. Hacker, M.A. Blank, J.M. Polank, S. R. Bloom, Peptides, 7, 5 (1986).

18. P. Miralles, E. Piero, P. Degano, A. Ramona, J. Marco, Diabetes, 39, 996 (1990).

19. T. Messel, H. Harling, G. Bottcher, A.H. Johnsen, J.J. Holst, Regulatory Peptides, 28, 161 (1990).

20. S.G. Gilby, J. Stephenson, D.J. O'Halloran, J.M. Burrin, S.R. Bloom, Diabetes, 38, 1114 (1989).

21. M.J. Dunne, M.J. Bullet, L. Guochong, C.B.-Wollheim, O.H. Petersen, EMBO, 8, 413 (1989).

22. E. Ekblad, R. Hakanson, F. Sundler, C. Wahlestedt, Br. J. Pharmacol, 86, 241 (1985): J.E.T., B. Brooks, T.J. McDonald, W. Barnett, F. Rostolanska, C. Yanaihara, A. Rokaeus, Peptides, 9, 1183 (1988).

23. B. Amiranoff, A.L. Servin, C.R. Rouyer-Fessard, A. Couvineau, K. Tatemoto and M. Luburthe, Endocrinology, 12, 284 (1987).

24. G. Skofitsch, M. Sills, D.M. Jacobowitz, Peptides, 7, 1029 (1986): T. Melander, T. Hokfelt, S. Nelsson, E. Brodin, Eur. J. Pharm., 124, 381 (1986).

25. C. Kohler, G. Hallmann, T. Melander, T. Hokfelt, E. Northeim, J. Chem. Neuro Anat. 2, 269 (1989).

26. B. Gallwitz, W.E. Shimdt, R. Schwarzhoff, W. Creutzfeldt, Biochem. and Biophys. Res. Communications, 172, 268 (1990): B. Amiranoff, A.M. Lorinet, N. Yanaihara, M. Laburthe, Eur. J. Pharm., 163, 205 (1989).

27. W.J. Rossowski, T.M. Rossowski, S. Zacharia, A. Ertan and D.H. Coy, Peptides, 11, 333, (1990).

## Claims

1. A polypeptide having the following amino acid sequence:- GWTLNSAGYLLGPHAVGNHRSFSDKNGLTS or a functional equivalent thereof being achieved by conservative substitutions selected from the following:
G, A; V, I, L, M; D, E; N, Q; S, T; K, R, H; and F, Y, W.

2. A polypeptide as claimed in claim 1 in which the polypeptide fragment has the following amino acid sequence:-

GWTLNSAGYLLGPHAVGNHRSFSDKNGLTS

3. A CDNA molecule encoding the peptide as claimed in claim 1 or 2, the cDNA molecule having a sequence substantially as shown in Figure 1 from nucleotide 97 to 186 or from nucleotide 97 to 145 or a functionally equivalent sequence.

4. A DNA molecule encoding human preprogalanin and galanin mRNA associated peptide, the DNA molecule having a sequence substantially as shown in Figure 1 or a functionally equivalent sequence.

5. A method of producing human galanin comprising a cell transformed with the DNA molecule of claim 3 or 4 under conditions which allow the expression of the DNA sequence and recovering the human galanin.

6. A method as claimed in claim 5 in which the cell is a bacterium.

7. The use of the peptide as claimed in claim 1 or claim 2 in the preparation of a medicament for modulating pancreatic activity.

8. The use of the peptide as claimed in claim 1 or claim 2 in the preparation of a medicament for the stimulation of the production of growth hormone.

9. The use of the peptide as claimed in claim 1 or claim 2 in the preparation of a medicament for attenuating cardiac vagal function.

10. A method of screening compounds for galanin agonist or antagonist activity comprising assessing the ability of the compound to compete with the peptide as claimed in claim 1 or claim 2 for binding to cell receptors and assessing the biological activity of the compounds which competitively bind.

11. The use of an antagonist to the peptide as claimed in claim 1 or claim 2 in the preparation of a medicament for increasing insulin secretion.

12. The use of an antagonist of the peptide as claimed in claim 1 or claim 2 in the preparation of a medicament for suppressing appetite.

13. The use of an agonist of the peptide as claimed in claim 1 or claim 2 in the preparation of a medicament for suppressing hyperinsulinaemia.

14. A peptide according to claim 1 or claim 2, for use in a method of testing the ability of an individual to produce growth hormone.

15. The use of the peptide of claim 1 or 2 or an agonist thereof in the manufacture of a composition for use in a method for testing the ability of an individual to produce growth hormone, which method comprises administering to the individual the said composition and measuring the levels of circulating growth hormone in the individual.

## Patentansprüche

1. Ein Polypeptid mit folgender Aminosäuresequenz: GWTLNSAGYLLGPHAVGNHRSFSDKNGLTS oder ein funktionelles Äquivalent desselben, das durch konservative Substitutionen, ausgewählt aus dem Nachfolgenden:
G, A; V, I, L , M; D, E; N, Q; S, T; K, R, H; und F, Y, W, erhalten wird.

2. Ein Polypeptid nach Anspruch 1, wobei das Polypeptidfragment die folgende Aminosäuresequenz aufweist:

GWTLNSAGYLLGPHAVGNHRSFSDKNGLTS.

3. Ein cDNA-Molekül, das für das Peptid nach Anspruch 1 oder 2 codiert, wobei das cDNA-Molekül eine Sequenz, wie im wesentlichen in Fig. 1 gezeigt, von Nukleotid 97 bis 186 oder von Nukleotid 97 bis 145 oder eine funktionell äquivalente Sequenz aufweist.

4. Ein DNA-Molekül, das für humanes Präprogalanin und für Galanin mRNA assoziiertes Peptid codiert, wobei das DNA-Molekül eine Sequenz aufweist, wie im wesentlichen in Fig. 1 gezeigt, oder eine funktionell äquivalente Sequenz.

5. Ein Verfahren zur Herstellung von humanem Galanin, das eine, mit dem DNA-Molekül nach Anspruch 3 oder 4 unter Bedingungen, die die Expression der DNA-Sequenz und das Gewinnen von humanem Galanin erlauben, transformierte Zelle umfaßt.

6. Ein Verfahren nach Anspruch 5, wobei die Zelle ein Bakterium ist.

7. Die Verwendung des Peptids nach Anspruch 1 oder 2 zur Herstellung eines Medikaments zur Regulierung der Pankreasaktivität.

8. Die Verwendung des Peptids nach Anspruch 1 oder 2 zur Herstellung eines Medikaments für die Stimulierung der Wachstumshormonproduktion.

9. Die Verwendung des Peptids nach Anspruch 1 oder 2 zur Herstellung eines Medikaments zur Dämpfung der Herzvagus-Funktion.

10. Ein Verfahren zum Testen von Verbindungen mit einer Wirksamkeit als Galaninagonisten oder -antagonisten, das das Bewerten der Fähigkeit der Verbindung, mit dem Peptid nach Anspruch 1 oder 2 um die Bindung an die Zellrezeptoren zu konkurrieren, und das Bewerten der biologischen Wirksamkeit der Verbindungen, die kompetitiv binden, umfaßt.

11. Die Verwendung eines Antagonisten für das Peptid nach Anspruch 1 oder 2 zur Herstellung eines Medikaments zur Verstärkung der Insulinsekretion.

12. Die Verwendung eines Antagonisten des Peptids nach Anspruch 1 oder 2 zur Herstellung eines Medikaments zur Unterdrückung des Nahrungsaufnahmeverlangens.

13. Die Verwendung eines Agonisten des Peptids nach Anspruch 1 oder 2 zur Herstellung eines Medikaments zur Unterdrückung der Hyperinsulinämie.

14. Ein Peptid nach Anspruch 1 oder 2 zur Verwendung bei einem Verfahren zur Überprüfung der Fähigkeit eines Individuums, Wachstumshormon herzustellen.

15. Die Verwendung des Peptids nach Anspruch 1 oder 2 oder eines Agonisten desselben zur Konfektionierung einer Zusammensetzung zur Verwendung bei einem Verfahren zur Überprüfung der Fähigkeit eines Individuums, Wachstumshormon herzustellen, wobei das Verfahren die Verabreichung der Zusammensetzung an ein Individuum und das Messen des zirkulierenden Wachstumshormonspiegels in dem Individuum umfaßt.

## Revendications

1. Polypeptide ayant la séquence d'acides aminés suivante : GWTLNSAGYLLGPHAVGNHRSFSDKNGLTS ou un équivalent fonctionnel de celle-ci étant réalisé par des substitutions conservatrices choisies parmi les suivantes :
G, A ; V, I, L, M ; D, E ; N, Q ; S, T ; K, R, H ; et F, Y, W.

2. Polypeptide selon la revendication 1, dans lequel le fragment de polypeptide présente la séquence d'acides aminés suivante :

GWTLNSAGYLLGPHAVGNHRSFSDKNGLTS

3. Molécule d'ADNc codant le peptide selon la revendication 1 ou 2, la molécule d'ADNc ayant une séquence sensiblement telle que représentée à la figure 1 du nucléotide 97 au nucléotide 186 ou du nucléotide 97 au nucléotide 145 ou une séquence fonctionnellement équivalente.

4. Molécule d'ADN codant le peptide associé à l'ARNm de la préprogalanine humaine et de la galanine, la molécule d'ADN ayant une séquence sensiblement telle que représentée en figure 1 ou une séquence fonctionnellement équivalente.

5. Procédé de préparation de galanine humaine comportant une cellule transformée par la molécule d'ADN de la revendication 3 ou 4 dans des conditions qui permettent l'expression de la séquence d'ADN et la récupération de la galanine humaine.

6. Procédé selon la revendication 5, dans lequel la cellule est une bactérie.

7. Utilisation du peptide selon la revendication 1 ou 2, pour la préparation d'un médicament pour réguler l'activité pancréatique.

8. Utilisation du peptide selon la revendication 1 ou 2, pour la préparation d'un médicament pour la stimulation de la production d'hormones de croissance.

9. Utilisation du peptide selon la revendication 1 ou 2, pour la préparation d'un médicament pour atténuer la fonction cardiaque vagale.

10. Procédé de tri de composés en ce qui concerne l'activité protagoniste ou antagoniste de galanine comprenant la prise en compte de la capacité du composé à entrer en compétition avec le peptide selon la revendication 1 ou 2 pour se lier à des récepteurs de cellules et à prendre en compte l'activité biologique des composés qui se lient de façon compétitive.

11. Utilisation d'un antagoniste au peptide selon la revendication 1 ou 2, pour la préparation d'un médicament pour augmenter la sécrétion d'insuline.

12. Utilisation d'un antagoniste du peptide selon les revendications 1 ou 2, pour la préparation d'un médicament pour supprimer l'appétit.

13. Utilisation d'un protagoniste du peptide selon les revendications 1 ou 2, pour la préparation d'un médicament pour supprimer l'hyperinsulinisme.

14. Peptide selon la revendication 1 ou 2, destiné à être utilisé dans un procédé de test de la capacité d'un individu à produire une hormone de croissance.

15. Utilisation du peptide selon la revendication 1 ou 2, ou d'un protagoniste de celui-ci, pour la fabrication d'une composition destinée à être utilisée dans un procédé de test de la capacité d'un individu à produire une hormone de croissance, ce procédé comprenant l'administration à l'individu de ladite composition et la mesure des niveaux d'hormone de croissance de l'individu.
